# EUROPÄISCHE PATENTSCHRIFT

(11) **EP 0 907 626 B1**
(45) Veröffentlichungstag und Bekanntmachung des Hinweises auf die Patenterteilung: **26.01.2000**
(21) Anmeldenummer: 97929308.1
(22) Anmeldetag: 30.06.1997
(51) Int. Cl.: C07C 17/02, C07C 19/045

(54) **VERFAHREN ZUR HERSTELLUNG VON 1,2-DICHLORETHAN DURCH DIREKTCHLORIERUNG**
PROCESS FOR PREPARING 1,2-DICHLOROETHANE BY DIRECT CHLORINATION
PROCEDE DE PREPARATION DE 1,2-DICHLOROETHANE PAR CHLORATION DIRECTE

(30) Priorität: 04.07.1996 DE 19626827; 09.10.1996 DE 19641562
(43) Veröffentlichungstag der Anmeldung: 14.04.1999
(73) Patentinhaber: Vinnolit Monomer GmbH & Co. KG, 85737 Ismaning (DE)
(72) Erfinder: SCHWARZMAIER, Peter, D-84556 Kastl (DE); MIELKE, Ingolf, D-84508 Burgkirchen (DE); GRUMANN, Helmut, D-84567 Perach (DE)
(74) Vertreter: Schuderer, Michael, Dr.
(86) Internationale Anmeldenummer: EP9703399
(87) Internationale Veröffentlichungsnummer: WO9801407

(56) Entgegenhaltungen:
- EP-A- 0 075 742
- EP-A- 0 471 987
- DE-A- 4 318 609
- US-A- 4 873 384

## Beschreibung

Die Erfindung betrifft ein Verfahren zur Herstellung von 1,2-Dichlorethan durch Direktchlorierung.

Die Herstellung von 1,2-Dichlorethan (im folgenden "EDC") durch Umsetzung von Ethylen mit Chlor, die allgemein als Direktchlorierung bezeichnet wird, verläuft unter Freisetzung von Reaktionswärme. Zur besseren Kontrolle der Reaktion und zum Abführen der Reaktionswärme dient üblicherweise umlaufendes flüssiges EDC. Hierzu wird aus dem Reaktionsraum flüssiges Reaktionsgemisch beziehungsweise Roh-EDC abgezogen und die Reaktionswärme über einen Wärmetauscher genutzt, beispielsweise zum Betreiben von Destillationskolonnen. Solche Verfahren sind beispielsweise aus EP-A-471 987 (ZA 91/6491), DE-A-4029314 und DE-A-41 33 810 bekannt. Aus diesen Druckschriften ist auch bekannt, durch geeignete Vorrichtungen wie statische Mischer eine besonders intensive Durchmischung der Reaktanden mit dem umlaufenden EDC sicherzustellen. US-4 873 384 beschreibt ein Verfahren zur Herstellung von EDC aus Ethylen und Chlor in flüssigem EDC wobei der Dampf des Reaktionsmediums dazu dient, einen Teil der latenten Wärme zurückzugewinnen.

Die Erfindung betrifft nun ein Verfahren zur Herstellung von EDC durch einspeisen von Ethylen und Chlor in umlaufendes EDC unter intensiver Durchmischung und Wärmerückgewinnung, das dadurch gekennzeichnet ist, daß man die Umsetzung bei 65 bis 125 °C und 0,5 bis 3,2 bar absolut durchführt, wobei man Druck und Temperatur so wählt, daß das Reaktionsgemisch siedet, und die Reaktionswärme aus dem Gasstrom abführt und einen Wärmetauscher zuführt.

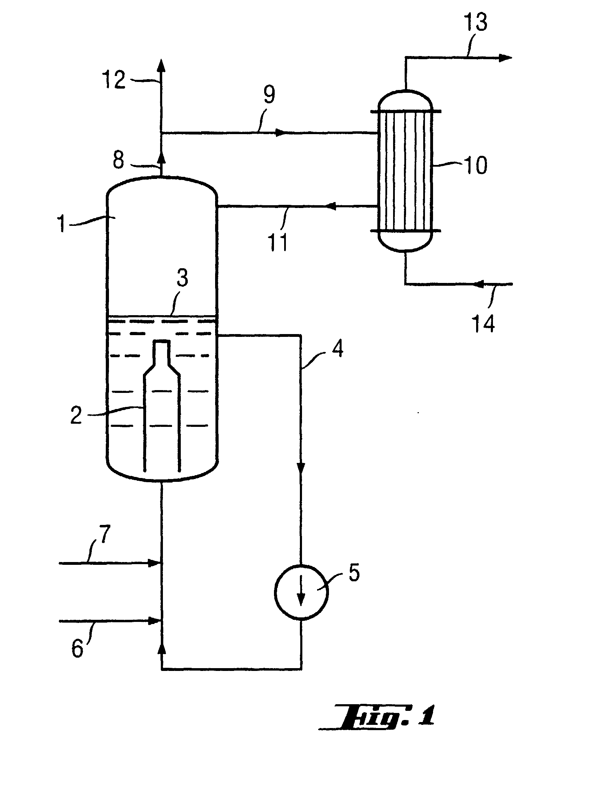

Die Erfindung betrifft weiterhin eine Vorrichtung zur Durchführung des Verfahrens, die schematisch in der Figur 1 wiedergegeben ist. In dieser Figur haben die Bezugsziffern die folgende Bedeutung:
1 = Reaktor
2 = Mischvorrichtung
3 = Grenze des flüssigen EDC
4 = Umlaufleitung für flüssiges EDC
5 = Pumpe
6 = Einspeisung für Chlor beziehungsweise Ethylen
7 = Einspeisung für Chlor beziehungsweise Ethylen
8 = Abzugsleitung für gasförmiges Reaktionsgemisch
9 = Leitung zum Wärmetauscher 10
10 = Wärmetauscher
11 = Rückleitung vom Wärmetauscher 10 zum Reaktor 1
12 = Leitung zur Destillationskolonne (nicht dargestellt)
13 = Leitung zum beziehungsweise vom Wärmeverbraucher
14 = Leitung zum beziehungsweise vom Wärmeverbraucher

Bevorzugte Ausgestaltungen des erfindungsgemäßen Verfahrens und der Vorrichtung werden im folgenden näher erläutert:

Eine Verfahrensvariante besteht darin, daß man aus dem Gasraum gasförmiges Reaktionsgemisch abzieht, das EDC in einem Wärmetauscher kondensiert und das flüssige EDC in den Reaktor zurückführt.

Eine weitere Ausgestaltung der Erfindung besteht darin, daß das gasförmige Reaktionsgemisch seitlich in eine Destillationskolonne eingespeist wird, aus der über Kopf inerte Gasanteile und nichtumgesetztes Ethylen, unterhalb der Einspeisungsstelle seitlich reines EDC abgezogen und aus dem Sumpf hochsiedende Nebenprodukte abgetrennt werden. Vorteilhaft kann diese Destillationskolonne mit der Reaktionswärme aus dem Gasraum des Reaktors betrieben werden. Die Temperatur im unteren Teil der Destillationskolonne ist hierbei etwas niedriger als die Temperatur im Reaktionsraum. Sie liegt beispielsweise bei 90 °C, wenn die Reaktion bei 105 °C durchgeführt wird.

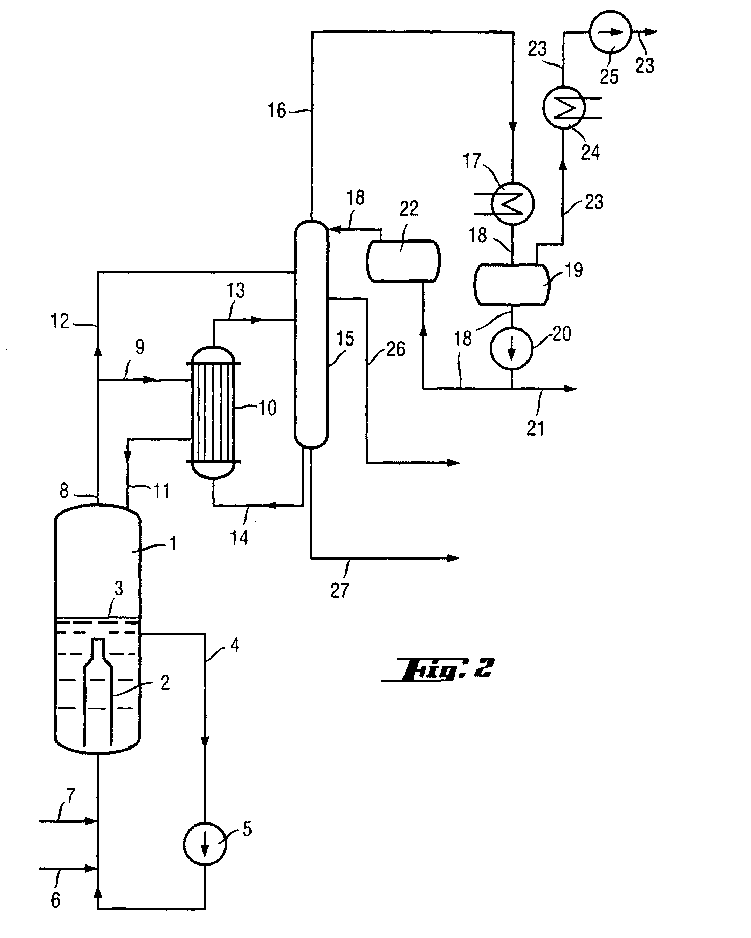

Eine geeignete Vorrichtung für diese Ausgestaltung der Erfindung ist in der Figur 2 wiedergegeben. In dieser haben die Bezugsziffern 1 bis 14 die vorstehend genannte Bedeutung, die übrigen stellen dar:
15 = Destillationskolonne
16 = Leitung für leichtflüchtige Produkte
17 = Kondensator
18 = Kreislaufleitung
19 = Rücklaufbehälter
20 = Pumpe
21 = Leitung zur Abführung leichtsiedender Produkte
22 = Trockner
23 = Leitung für Abgas
24 = Kondensator
25 = Pumpe
26 = Leitung für EDC
27 = Leitung für hochsiedende Produkte

Die leichtflüchtigen Produkte gelangen über Kopf der Destillationskolonne 15 durch die Leitung 16 und den Kondensator 17 über die Kreislaufleitung 18 in den Behälter 19 (Rücklaufbehälter). Kondensierte flüssige Produkte gelangen weiter über die Kreislaufleitung 18 und eine Pumpe 20 in einen Trockner 22, der verhindert, daß sich eingeschlepptes Wasser in diesem Kreislauf anreichert und Korrosion verursacht. Über eine Leitung 21 können leichtsiedende Produkte getrennt ausgebracht werden.

Aus dem Behälter 19 gelangen gasförmige Produkte, im wesentlichen nichtumgesetztes Ethylen und inerte Anteile, über einen weiteren Kondensator 24 und eine Pumpe 25 zur Abgasverwertung.

Der Trockner 22 kann in üblicher Weise ausgestaltet sein und beispielsweise nach bekannten physikalischen und/oder chemischen Methoden funktionieren. Wenn der Trockner 22 ein Trockenmittel enthält, sind chemische Trocknungsmittel wie Phosphorpentoxid oder physikalische Trocknungsmittel wie Molekularsiebe oder Kieselgele geeignet. Vorteilhaft erfolgt die Trocknung wie in der US-A-5 507 920 angegeben.

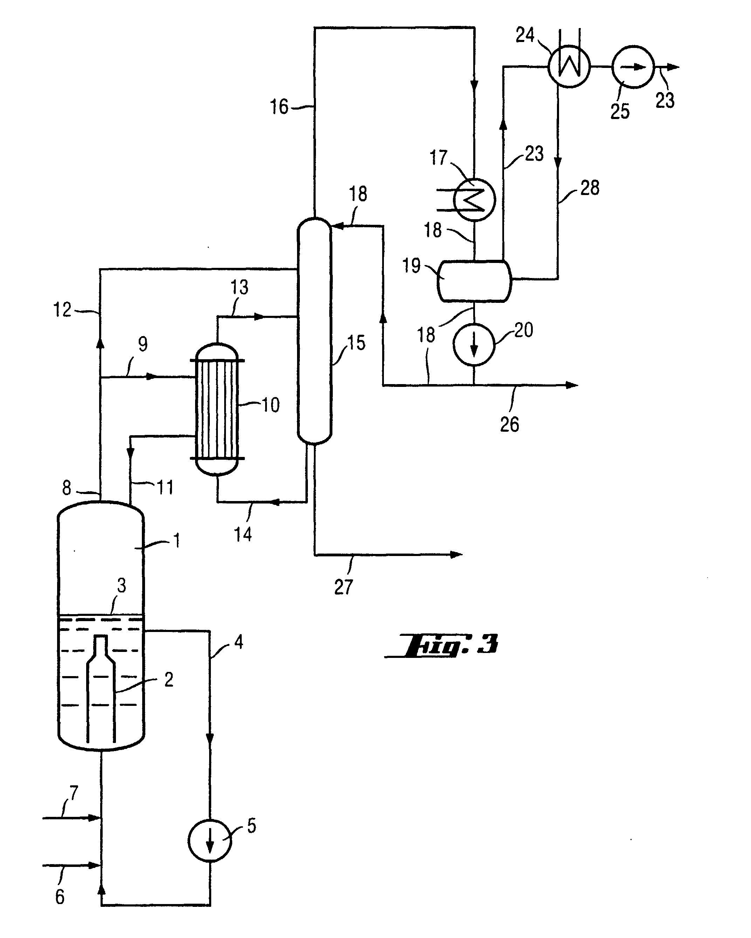

In einer anderen Ausgestaltung der Erfindung wird die Destillationskolonne unter verminderem Druck betrieben. Diese Ausführungsform ist in der Figur 3 dargestellt. Hierin haben die Bezugsziffern 1 bis 21 (der Trockner 22 entfällt) und 23 bis 27 die vorstehend genannten Bedeutungen und 28 ist eine Rücklaufleitung vom Kondensator 24 zum Behälter 19.

Hierbei steht der Behälter 19 unter stärker vermindertem Druck als die Kolonne 15 (beispielsweise in der Kolonne 15 0,8 bar absolut, im Behälter 19 0,26 bar absolut). Die Druckregelung erfolgt hierbei durch eine oder mehrere Pumpen, beispielsweise die Pumpe 25 (mit entsprechenden Ventilen, die in der Figur nicht dargestellt sind). Bei dieser Ausführungsform werden im Behälter 19 die über den Kühler 17 eintreffenden Produkte entspannt. Die Gasphase gelangt über die Leitung 23 in den Kühler 24, aus dem verflüssigte Produkte über die Leitung 28 zurück zum Behälter 19 fließen. Die Flüssigphase - reines EDC - wird nach der Pumpe 20 getrennt in den Produktstrom (über Leitung 26) und den Rücklauf strom 18.

Das Verfahren wird mit den üblichen Katalysatoren durchgeführt. Geeignet sind Kombinationen aus Lewissäuren wie Eisen-(III)-chlorid und Halogeniden von Metallen der ersten oder zweiten Hauptgruppe des Periodensystems der Elemente, vor allem Natriumchlorid, in den verschiedensten Molverhältnissen (NL-A-6901398, US-A-4 774 373 oder DE-A-41 03 281) und insbesondere mit dem Katalysatorsystem nach WO-A-94/17019 (ZA 94/0535), bei welchem während der gesamten Umsetzung das Molverhältnis von Natriumchlorid zu Eisen-(III)-chlorid unter 0,5, vorzugsweise im Bereich 0,45 bis 0,3, bleibt. Bei diesem Verfahren wird das EDC in so hoher Reinheit erhalten, daß besonders lange Standzeiten der Wärmetauscher erreicht werden.

Die erfindungsgemäße Durchführung des Verfahrens bringt eine Reihe von Vorteilen mit sich:

Die Reaktion kann sehr sicher geführt und jederzeit gut kontrolliert werden. Hierdurch wird es möglich, die Reaktionstemperatur niedrig zu halten, was die Bildung von Nebenprodukten unterdrückt. Dadurch, daß die Reaktionswärme aus dem gasförmigen Reaktionsgemisch abgeführt wird, können die Wärmetauscher, zum Beispiel Umlaufverdampfer, klein dimensioniert werden, da die Kondensationswärme des EDC mitgenutzt wird. Von Vorteil ist auch, daß die Wärmetauscher nicht durch mitgerissenen Katalysator und hochsiedende Nebenprodukte verschmutzt werden.

Die Ausnutzung der Reaktions- und Kondensationswärme ist sehr effektiv und erlaubt eine Vielzahl von konstruktiven Ausgestaltungen des Verfahrens. Der oder die Wärmetauscher können unmittelbar benachbart zum Reaktor angeordnet werden und auch die die Wärme nutzenden Apparaturen können ihrerseits räumlich unmittelbar benachbart an beziehungsweise um den oder die Wärmetauscher angebaut werden. Hierdurch können konstruktiver Aufwand und Wärmeverluste durch lange Leitungen vermieden werden und wertvoller Platz in der Anlage gespart werden.

Bei den vorstehend genannten Ausführungsformen der Erfindung, bei denen inerte Gasanteile und nichtumgesetztes Ethylen entfernt werden, kann das Ethylen in bekannter Weise von den inerten Anteilen abgetrennt und in den Prozeß zurückgeführt werden. Solche Gasanteile wie Sauerstoff oder Stickstoff werden beispielsweise durch das Chlor eingeschleppt, wobei hier der Sauerstoff bei einer Volumenkonzentration unterhalb der Explosionsgrenze (3 %) als inert gilt. Die Abgasrückführung bei der Direktchlorierung ist in WO-A-96/03361 (ZA 95/6058) beschrieben.

Die Durchführung der Reaktion erfolgt in an sich bekannter Weise, wobei auf die vorstehend genannten Druckschriften, auch hinsichtlich der apparativen Einzelheiten, verwiesen wird.

Das erfindungsgemäße Verfahren wird in den folgenden Beispielen näher erläutert.

### Beispiel 1 (Figuren 1 und 2)

In einem Direktchlorierungsreaktor 1 mit einem statischen Mischer 2 werden über die Leitung 6 Chlor und über die Leitung 7 Ethylen eingespeist. Der Reaktor wird mit flüssigem EDC bis zum Flüssigkeitsstand 3 befüllt und über die Leitung 4 und die Pumpe 5 umgepumpt. Das aus dem Dampfraum des Reaktors über die Leitung 8 austretende Gasgemisch (im wesentlichen EDC, aber auch Spuren von nichtumgesetztem Ethylen, Sauerstoff, Stickstoff und leichter als EDC siedenden Komponenten) wird zum größten Teil (etwa 85 %) über die Leitung 9 einem Kolonnenaufheizer 10 (Wärmetauscher) zugeführt, dort kondensiert und über die Leitung 11 wieder in den Reaktor 1 geleitet. Die Kondensationsenergie wird über die Leitungen 13 und 14 der Destillationskolonne 15 zugeführt beziehungweise von dieser abgeführt.

Der kleinere Teil des Gasgemisches wird über die Leitung 12 in die Destillationskolonne 15 seitlich eingespeist, in der nichtumgesetztes Ethylen, Sauerstoff, Stickstoff sowie Spuren von leichter siedenden Nebenprodukten wie Ethylchlorid und Wasser über Kopf (Leitung 16) abgetrennt werden. Das Rein-EDC wird aus der Kolonne 15 über die Leitung 26 (unterhalb der Einspeisung der Leitung 12) abgezogen.

Nichtkondensierbare Stoffe wie Ethylen, Sauerstoff und Stickstoff gelangen über die Leitung 16, den Kondensator 17, die Leitung 18, den Behälter 19 und die Leitung 23 zu einem Abgaskondensator 24, dann zum Kompressor 25, der sie zu einer Abgasverwertungsanlage drückt.

Kondensierbare Stoffe wie leichter siedende Nebenprodukte und ein azeotropes Gemisch aus EDC und Wasser gelangen ebenfalls zunächst über die Leitung 16, den Kondensator 17 und die Kreislaufleitung 18 in den Rücklaufbehälter 19, aber von dort über die Förderpumpe 20 zum Trockner 22, der verhindert, daß sich das in Spuren eingetragene Wasser am Kolonnenkopf anreichert. Das getrocknete Kondensat fließt dann über die Kreislaufleitung 18 in die Destillationskolonne 15.

### Beispiel 2 (Figur 3)

Man verfährt nach Beispiel 1, erster Absatz, dann jedoch wie folgt:

Der kleinere Teil des Gasgemisches wird über die Leitung 12 in die Destillationskolonne 15 eingespeist. Reines EDC sowie nichtumgesetztes Ethylen, Sauerstoff und Stickstoff und Spuren von leichter siedenden Komponenten über die Leitung 16 gelangen zum EDC-Kondensator 17 und dann über die Leitung 18 zum Rücklaufbehälter 19. Mit einer (einzigen) Vakuumpumpe 25 werden in der Kolonne 15 0,8 bar absolut und im Rücklaufbehälter 19 0,26 bar absolut eingestellt, um das nichtumgesetzte, im EDC gelöste Ethylen sowie den Sauerstoff und Stickstoff abzutrennen. Im Abgaskühler 24 wird bei + 1 °C weiteres EDC kondensiert, und das Abgas wird über die Leitung 23 einer Abgasverwertungsanlage zugeführt. Reines EDC aus dem Rücklaufbehälter 19 wird über die Leitung 26 einem EDC-Spaltofen zugeführt.

## Patentansprüche

1. Verfahren zur Herstellung von 1,2-Dichlorethan (EDC) durch Einspeisen von Ethylen und Chlor in umlaufendes EDC unter intensiver Durchmischung und Wärmerückgewinnung, dadurch gekennzeichnet, daß man die Umsetzung bei einer Temperatur von 65 bis 125 °C und einem Druck von 0,5 bis 3,2 bar absolut durchführt, wobei Druck und Temperatur so gewählt werden, daß das Reaktionsgemisch siedet, und die Reaktionswärme aus dem Gasraum abgeführt und mindestens einem Wärmetauscher zugeführt wird.

2. Verfahren nach Anspruch 1, dadurch gekennzeichnet, daß aus dem Gasraum ein Teil des gasförmigen Reaktionsgemisches abgezogen, das EDC in einem Wärmetauscher kondensiert und das flüssige EDC in den Reaktor zurückgeführt wird.

3. Verfahren nach Anspruch 1 oder 2, dadurch gekennzeichnet, daß ein Teil des gasförmigen Reaktionsgemisches seitlich in eine Destillationskolonne eingespeist wird, aus der über Kopf inerte Gasanteile und nichtumgesetzes Ethylen, unterhalb der Einspeisestelle seitlich reines EDC abgezogen und aus dem Sumpf hochsiedende Nebenprodukte abgetrennt werden.

4. Verfahren nach Anspruch 3, dadurch gekennzeichnet, daß die Destillationskolonne mit der Reaktionswärme aus dem Gasraum des Reaktors betrieben wird.

5. Verfahren nach einem oder mehreren der vorhergehenden Ansprüche, dadurch gekennzeichnet, daß die intensive Durchmischung mit einem statischen Mischer erreicht wird.

6. Verfahren nach einem oder mehreren der vorhergehenden Ansprüche, dadurch gekennzeichnet, daß die Reaktion mit einem Katalysatorsystem aus einer Lewissäure und einem Halogenid der ersten und zweiten Gruppe des Periodensystems durchgeführt wird.

7. Verfahren nach Anspruch 6, dadurch gekennzeichnet, daß als Katalysator Natriumchlorid und Eisen-(III)-chlorid im Molverhältnis unter 0,5 eingesetzt wird.

8. Vorrichtung zur Durchführung des Verfahrens nach den Ansprüchen 1 bis 7, gekennzeichnet durch einen Reaktor (1), eine Mischvorrichtung (2), eine Grenze des flüssigen EDC (3), eine Umlaufleitung für flüssiges EDC (4), eine Pumpe (5), Einspeisungen für Chlor beziehungsweise Ethylen (6, 7), eine Abzugsleitung für gasförmiges Reaktionsgemisch (8), eine Leitung (9) zum Wärmetauscher (10), einen Wärmetauscher (10), eine Rückleitung (11) vom Wärmetauscher (10) zum Reaktor (1), eine Leitung (12) zu einer Destillationskolonne und Leitungen (13, 14) zu beziehungsweise von einem Wärmeverbraucher.

9. Vorrichtung nach Anspruch 8, gekennzeichnet durch eine Destillationskolonne (15), eine Leitung für leichtflüchtige Produkte (16), einen Kondensator (17), eine Kreislaufleitung (18), einen Rücklaufbehälter (19), eine Pumpe (20), eine Leitung zur Abführung leichtsiedender Produkte (21), einen Trockner (22), eine Leitung für Abgas (23), einen Kondensator (24), eine Pumpe (25), eine Leitung für EDC (26) und eine Leitung für hochsiedende Produkte (27).

10. Vorrichtung nach Anspruch 9, bei der der Trockner (22) entfällt und eine Rücklaufleitung (28) vom Kondensator (24) zum Behälter (19) vorgesehen ist.

## Claims

1. Process for preparing 1,2-dichloroethane (EDC) by feeding ethylene and chlorine into circulating EDC with intensive mixing and heat recovery, characterized in that the reaction is carried out at a temperature of from 65 to 125°C and at a pressure of from 0.5 to 3.2 bar absolute, pressure and temperature being chosen such that the reaction mixture boils, and the heat of reaction is conducted away from the gas space and is supplied to at least one heat exchanger.

2. Process according to Claim 1, characterized in that part of the gaseous reaction mixture is taken off from the gas space, the EDC is condensed in a heat exchanger and the liquid EDC is passed back to the reactor.

3. Process according to Claim 1 or 2, characterized in that part of the gaseous reaction mixture is fed at the side into a distillation column from which inert gas fractions and unreacted ethylene are taken off from the top, pure EDC is taken off at the side below the infeed point, and high-boiling byproducts are separated off from the bottom.

4. Process according to Claim 3, characterized in that the distillation column is operated with the heat of reaction from the gas space of the reactor.

5. Process according to one or more of the preceding claims, characterized in that the intensive mixing is effected with a static mixer.

6. Process according to one or more of the preceding claims, characterized in that the reaction is carried out with a catalyst system comprising a Lewis acid and a halide from the first or second group of the Periodic Table of the Elements.

7. Process according to Claim 6, characterized in that the catalyst employed comprises sodium chloride and iron(III) chloride in a molar ratio of below 0.5.

8. Apparatus for carrying out the process according to Claims 1 to 7, characterized by a reactor (1), a mixing device (2), a limit of the liquid EDC (3), a circulation line for liquid EDC (4), a pump (5), infeed points for chlorine and ethylene respectively (6, 7), an offtake line for gaseous reaction mixture (8), a line (9) to the heat exchanger (10), a heat exchanger (10), a return line (11) from the heat exchanger (10) to the reactor (1), a line (12) to a distillation column and lines (13, 14) to and from, respectively, the heat consumer unit.

9. Apparatus according to Claim 8, characterized by a distillation column (15), a line for volatile products (16), a condenser (17), a circuit line (18), a return flow vessel (19), a pump (20), a line for taking off low-boiling products (21), a drier (22), a line for off-gas (23), a condenser (24), a pump (25), a line for EDC (26) and a line for high-boiling products (27).

10. Apparatus according to Claim 9, in which the drier (22) is omitted and a return flow line (28) from the condenser (24) to the container (19) is provided.

## Revendications

1. Procédé de préparation de 1,2-dichloroéthane (EDC) par alimentation d'éthylène et de chlore dans de l'EDC circulant sous mélange intime intensif et récupération de chaleur, caractérisé en ce qu'on effectue la transformation à une température de 65 à 125°C et à une pression absolue de 0,5 à 3,2 bars, la pression et la température étant sélectionnées de sorte que le mélange réactionnel est à ébullition, et que la chaleur réactionnelle est évacuée hors du volume de gaz et est conduite à au moins un échangeur de chaleur.

2. Procédé suivant la revendication 1, caractérisé en ce qu'on soutire du volume de gaz une partie du mélange réactionnel gazeux, l'EDC est condensé dans un échangeur de chaleur et l'EDC liquide est ramené dans le réacteur.

3. Procédé suivant la revendication 1 ou 2, caractérisé en ce qu'une partie du mélange réactionnel gazeux est alimentée latéralement dans une colonne de distillation, hors de laquelle on soutire, par la tête, des proportions de gaz inertes et de l'éthylène non transformé, sous le point d'alimentation, latéralement, de l'EDC pur et on sépare, par le bas, des produits secondaires à haut point d'ébullition.

4. Procédé suivant la revendication 3, caractérisé en ce que la colonne de distillation fonctionne avec la chaleur réactionnelle provenant du volume de gaz du réacteur.

5. Procédé suivant l'une ou plusieurs des revendications précédentes, caractérisé en ce que le mélange intime intensif est obtenu avec un mélangeur statique.

6. Procédé suivant l'une ou plusieurs des revendications précédentes, caractérisé en ce que la réaction est effectuée avec un système de catalyseur à partir d'un acide de Lewis et d'un halogénure du premier et deuxième groupe du système périodique.

7. Procédé suivant la revendication 6, caractérisé en ce qu'on utilise, comme catalyseur, du chlorure de sodium et du chlorure de fer (III) dans le rapport molaire inférieur à 0,5.

8. Dispositif pour la réalisation du procédé suivant les revendications 1 à 7, caractérisé par un réacteur (1), un dispositif de mélange (2), une limite de l'EDC liquide (3), une conduite de circulation pour l'EDC liquide (4), une pompe (5), des alimentations pour du chlore, ou, selon le cas, de l'éthylène (6, 7), une conduite de soutirage pour le mélange réactionnel gazeux (8), une conduite (9) vers l'échangeur de chaleur (10), un échangeur de chaleur (10), une conduite de retour (11) de l'échangeur de chaleur (10) vers le réacteur (1), une conduite (12) vers une colonne de distillation, et des conduites (13, 14) vers un consommateur de chaleur ou, selon le cas, depuis celui-ci.

9. Dispositif suivant la revendication 8, caractérisé par une colonne de distillation (15), une conduite pour des produits facilement volatils (16), un condenseur (17), une conduite de circulation (18), un réservoir de retour (19), une pompe (20), une conduite pour l'évacuation de produits à bas point d'ébullition (21), un sécheur (22), une conduite pour gaz d'échappement (23), un condenseur (24), une pompe (25), une conduite pour EDC (26) et une conduite pour des produits à haut point d'ébullition (27).

10. Dispositif suivant la revendication 9, dans lequel le sécheur (22) est supprimé et une conduite de retour (28) du condenseur (24) vers le réservoir (19) est prévue.
